(19) Europäisches Patentamt
European Patent Office
Office européen des brevets

(11) Veröffentlichungsnummer: **0 068 219**
**B1**

(12) **EUROPÄISCHE PATENTSCHRIFT**

(45) Veröffentlichungstag der Patentschrift: **29.08.84**

(21) Anmeldenummer: **82105100.0**

(22) Anmeldetag: **11.06.82**

(51) Int. Cl.³: **C 07 C 51/06,** C 07 C 57/32, C 07 C 57/58, C 07 C 63/36, C 07 C 87/123, C 07 C 87/127, C 07 C 85/20, C 07 D 213/803, C 07 C 57/30

(54) **Verfahren zur Herstellung von Carbonsäuren und N-tert. Alkylaminen.**

(30) Priorität: **23.06.81 DE 3124652**
**27.03.82 DE 3211326**

(43) Veröffentlichungstag der Anmeldung: **05.01.83 Patentblatt 83/1**

(45) Bekanntmachung des Hinweises auf die Patenterteilung: **29.08.84 Patentblatt 84/35**

(84) Benannte Vertragsstaaten: **DE GB**

(56) Entgegenhaltungen: **DE - A - 2 164 239**

(73) Patentinhaber: **BAYER AG, Konzernverwaltung RP Patentabteilung, D-5090 Leverkusen 1 Bayerwerk (DE)**

(72) Erfinder: **Bonse, Gerhard, Dr., Wolfskaul 3, D-5000 Köln 80 (DE)**
Erfinder: **Marzolph, Gerhard, Dr., Roggendorfstrasse 65, D-5000 Köln 80 (DE)**
Erfinder: **Blank, Heinz Ulrich, Dr., Am Geusfelde 35, D-5068 Odenthal (DE)**

**EP 0 068 219 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

**Beschreibung**

Die vorliegende Erfindung betrifft ein Verfahren zur gleichzeitigen Herstellung von Carbonsäuren und N-tert.-Alkylaminen durch alkalische Druckhydrolyse von N-tert.-Alkylcarbonsäureamiden.

Carbonsäuren können durch Hydrolyse der entsprechenden Nitrile dargestellt werden. So wird z. B. Phenylessigsäure durch alkalische oder saure Hydrolyse von Benzylcyanid hergestellt (Ullmanns Enzyklopädie der technischen Chemie, 4. Auflage, 1976, Band 11, Seite 71; Organic Syntheses, Coll. Vol. 1, 436). Bei diesen Hydrolyseverfahren geht der Stickstoff der Nitrilgruppe als Ammoniak oder Ammoniumsalz verloren.

N-t-Alkylamine können durch Hydrolyse von ausgewählten N-t-Alkylamiden hergestellt werden. Dazu müssen alkalische Hydrolysebedingungen angewendet werden, denn unter sauren Bedingungen spalten diese Amide in Olefin, Ammoniak und Carbonsäure (J. Am. Chem. Soc. 70 (1948), 4046).

Die alkalische Hydrolyse von N-tert.-Alkylamiden erfordert jedoch drastische Reaktionsbedingungen. So beschreibt US 2 548 585 die Hydrolyse von N-t-Butylharnstoff bzw. von N,N'-Di-t-butylharnstoff mit mindestens 2 Mol NaOH pro Mol des Einsatzmaterials in Ethoxyethanol als Lösungsmittel, wobei das abgespaltene t-Butylamin aus dem Reaktionsmedium direkt abdestilliert wird.

In J. Am. Chem. Soc. 70 (1948), 4048 ist beschrieben, daß N-tert.-Alkylformamide durch Erhitzen unter Rückfluß mit 20—32%iger NaOH unter Freisetzung des Amins hydrolysiert werden können. Dieses Verfahren wird auch in der DE-AS 2 236 040 zur Hydrolyse von N-tert.-Alkylformamiden angewendet.

Die Hydrolyse von Amiden höherer Carbonsäuren verläuft hingegen sehr viel schlechter. So lehrt Zabicki in Chemistry of Amides S. 824 ff (Interscience 1970), daß die Geschwindigkeit der alkalischen Hydrolyse von Amiden durch Verzweigung am $\alpha$-C-Atom des Carbonsäurerestes stark verlangsamt wird. Noch stärker ist der Einfluß der Verzweigung am $\alpha$-C-Atom des Aminrestes. So sinkt die Hydrolysegeschwindigkeit bei Acetamiden von Acetamid zum N-Methylacetamid auf 1/18 ab, die Hydrolysegeschwindigkeit des N-t-Butylacetamids war so klein, daß sie nicht bestimmt werden konnte.

Auch Scholl beschreibt in Liebigs Annalen der Chemie 338, 16 (1905), daß die Hydrolyse von N-t-Butylacetamid sehr drastische Bedingungen erfordert. Nach J. Am. Chem. Soc. 70 (1948), 4048 gelingt die Hydrolyse des N-t-Butylacetamids mit KOH in Glykol bei Rückflußbedingungen (Kp von Glykol: 197° C) selbst nach 48 h Reaktionszeit nur mit 27% Ausbeute an t-Butylamin.

Die rumänische Patentanmeldung 55 714 (zitiert nach C. A. 80, 107972 x) beschreibt die Hydrolyse von N-Butylacetamid mit Kaliumhydroxid in Ethylenglykol.

Die Hydrolyse des N-t-Octylacetamids mit KOH in Glykol liefert nach 2 Tagen Erhitzen unter Rückfluß das Amin in nur 62% Ausbeute (J. Am. Chem. Soc. 70 (1948) 4048).

Bei allen bisher genannten Verfahren können zwar die t-Alkylamine gewonnen werden, jedoch geht die Säurekomponente in der Regel im Abwasser verloren. Außerdem verlangt die Arbeitsweise in polaren hochsiedenden Lösungsmitteln wie Glykol besondere Anstrenungen zur Abwasserreinigung.

In DE-OS 2 164 239 wird die alkalische Spaltung von Propionsäure- und Acrylsäure-N-t-alkylamiden in NaOH oder KOH in Methanol oder Methanol/Wasser-Gemischen als Reaktionsmedium bei Temperaturen von 150 bis 250° C beschrieben. Das Verfahren hat den Nachteil, daß das bei der Aufarbeitung anfallende Gemisch aus Methanol, Wasser und t-Butylamin nur durch einen hohen Destillationsaufwand und nachträgliche Trocknung des Amins zu reinem t-Butylamin aufgearbeitet werden kann. Ebenso bereitet die Wiedergewinnung des Methanols aus dem Reaktionsansatz einen erhöhten Aufwand.

In der japanischen Patentanmeldung 50-95 210 wird die Hydrolyse des Thio-bispropionsäure-N-t-butylamids mit 13%iger NaOH bei 165 bis 185° C beschrieben. Die Nacharbeitung der Versuche zeigte aber, daß man nach diesem Verfahren höchstens 70—75% t-Butylamin und 50% Thiobispropionsäure isolieren kann. Es setzt eine tiefgreifende Zersetzung des Moleküls ein, die bei Temperaturen oberhalb 200° C die Isolierung von Thiobispropionsäure verhindert.

Ferner ist aus J. Am. Chem. Soc. 70 (1948), 4048 bekannt, daß N-tert.-Alkylamide bei erhöhter Temperatur, zum Beispiel bei der Destillation, in das zugrunde liegende Nitril, in Olefin und Wasser gespalten werden können.

Auf Grund der geschilderten Fakten war es überraschend und nicht vorhersehbar, daß man N-t-Alkylamide bei einer Temperatur von mindestens 200° C mit Alkalihydroxid in Wasser als Reaktionsmedium in glatter Reaktion und mit guten Ausbeuten zur zugrundeliegenden Carbonsäure und dem t-Alkylamin hydrolysieren kann.

Es wurde nunmehr ein Verfahren zur gleichzeitigen Herstellung von Carbonsäuren und N-tert.-Alkylaminen gefunden, das dadurch gekennzeichnet ist, daß man ein Carbonsäure-N-t-alkylamid der Formel

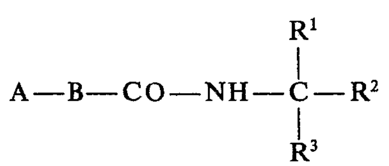

(I)

in der

A für einen gegebenenfalls substituierten Phenyl-, Naphthyl- oder Pyridylrest oder den aliphatischen Rest

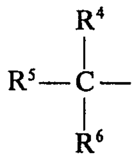

steht, wobei $R^4$, $R^5$ und $R^6$ gleich oder verschieden sind und unabhängig voneinander Wasserstoff oder geradkettige oder verzweigte Alkylgruppen mit 1 bis 8 Kohlenstoffatomen bedeuten, wobei weiterhin $R^4$ und $R^5$ gemeinsam einen aliphatischen Ring von 4 bis 8 Ringgliedern bilden können,

B für eine Kohlenstoff-Einfachbindung, für einen aliphatischen Rest mit 1 bis 6 Kohlenstoffatomen oder einen cycloaliphatischen Rest mit 4 bis 8 Kohlenstoffatomen steht, wobei jedes Kohlenstoffatom unabhängig von den anderen mit geradkettigen oder verzweigten $C_1$—$C_4$-Alkylgruppen substituiert sein kann, und

$R^1$, $R^2$, $R^3$ gleich oder verschieden sind und unabhängig voneinander geradkettige oder verzweigte Alkylgruppen mit 1 bis 8 Kohlenstoffatomen bedeuten, wobei weiterhin $R^1$ und $R^2$ gemeinsam einen aliphatischen Ring von 4 bis 8 Ringgliedern bilden können oder $R^1$ ein gegebenenfalls substituierter Phenylrest sein kann,

mit 5- bis 50gew.-%igem wäßrigem Alkalihydroxid in einer Menge von 1,0 bis 1,3 Mol pro Mol des Amids bei 200 bis 350° C unter erhöhtem Druck umsetzt.

Vorzugsweise bedeutet A einen gegebenenfalls substituierten Phenyl-, Naphthyl- oder Pyridylrest, besonders bevorzugt einen gegebenenfalls substituierten Phenyl- oder Naphthylrest, ganz besonders bevorzugt den gegebenenfalls substituierten Phenylrest.

Bevorzugt bedeutet B eine Kohlenstoff-Einfachbindung oder eine $CH_2$-Gruppe, die mit geradkettigen und/oder verzweigten $C_1$—$C_4$- Alkylgruppen substituiert sein kann, besonders bevorzugt eine $CH_2$-Gruppe.

Bevorzugt bedeuten $R^1$, $R^2$, $R^3$ unabhängig voneinander die Methyl- oder Ethylgruppe.

Als Substituenten für den Phenyl- oder Naphthylrest kommt Halogen, wie Fluor, Chlor oder Brom, bevorzugt Fluor oder Chlor, geradkettiges oder verzweigtes $C_1$—$C_6$-Alkyl, bevorzugt Methyl oder Ethyl, $C_4$—$C_8$-Cycloalkyl, bevorzugt $C_5$—$C_6$-Cycloalkyl oder Phenyl in Frage.

Als Substituenten für den Pyridylrest kommt geradkettiges oder verzweigtes $C_1$—$C_6$-Alkyl, bevorzugt Methyl oder Ethyl in Frage.

Daraus ergeben sich beispielsweise bevorzugt in das erfindungsgemäße Verfahren einsetzbare Carbonsäure-N-tert.-alkylamide der Formel

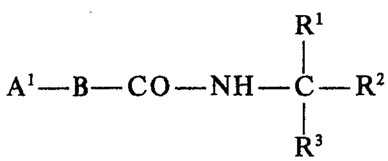
(II)

in der

$A^1$ den gegebenenfalls substituierten Phenyl-, Naphthyl- oder Pyridylrest bedeutet und
B, $R^1$, $R^2$ und $R^3$ die oben angegebene Bedeutung haben.

Weitere bevorzugt einsetzbare Carbonsäure-N-tert.-alkylamide sind solche der Formel

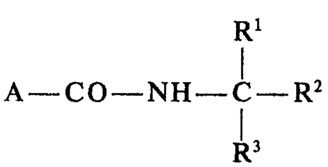
(III)

in der

A, $R^1$, $R^2$ und $R^3$ die oben angegebene Bedeutung haben.

Besonders bevorzugt einsetzbare Carbonsäure-N-tert.-alkylamide sind solche der Formel

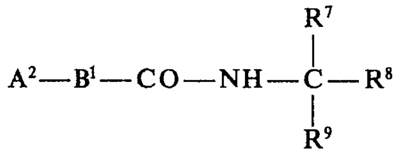

(IV)

in der

$A^2$ den gegebenenfalls substituierten Phenylrest,
$B^1$ eine Kohlenstoff-Einfachbindung oder eine $CH_2$-Gruppe, die mit geradkettigen und/oder verzweigten $C_1-C_4$-Alkylgruppen substituiert sein kann, und
$R^7$, $R^8$ und $R^9$ unabhängig voneinander Methyl oder Ethyl bedeuten.

Weitere besonders bevorzugte Carbonsäure-N-tert.-alkylamide sind solche der Formel

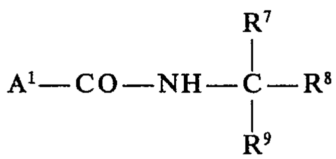

(V)

in der

$A^1$, $R^7$, $R^8$ und $R^9$ die oben angegebene Bedeutung haben.

Ganz besonders bevorzugt sind Carbonsäure-N-tert.-alkylamide der Formel

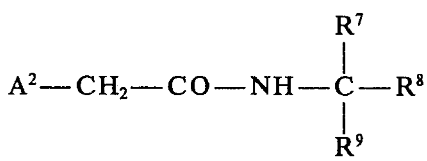

(VI)

in der

$A^2$, $R^7$, $R^8$ und $R^9$ die obengenannte Bedeutung haben.

Beispielhaft unter der Formel (VI) sei das Phenylessigsäure-N-tert.-butylamid genannt.

Die erfindungsgemäß einsetzbaren N-t-Alkylamide sind bekannt oder können nach der als Ritter-Reaktion bekannten Umsetzung aus den Carbonsäurenitrilen mit tert.-Alkoholen, t-Alkylethern, t-Alkylestern oder Olefinen in Gegenwart von starken Säuren hergestellt werden (Houben—Weyl, Methoden der organischen Chemie, Band 11/1, Seiten 994 bis 1000 (1957); Organic Reactions, Vol. 17, Seiten 215 bis 324; Russian Chemical Review 29, 334 (1960)). Sie werden daher im folgenden als Ritter-Amide bezeichnet.

Als Alkalihydroxide seien beispielsweise die Hydroxide aller Metalle der ersten Hauptgruppe des Periodensystems (Mendelejew), bevorzugt jedoch Natriumhydroxid oder Kaliumhydroxid, genannt. Das Alkalihydroxid wird in Form einer 5- bis 50gew.-%igen wäßrigen Lösung, bevorzugt einer 10- bis 35gew.-%igen wäßrigen Lösung, eingesetzt.

Als Menge des Alkalihydroxids sei beispielsweise 1,0 bis 1,3 Mol, bevorzugt 1,0 bis 1,15, besonders bevorzugt 1,0 bis 1,05 Mol pro Mol des Amids genannt. Selbstverständlich kann das Ritter-Amid auch mit Alkalihydroxidmengen außerhalb der genannten Bereiche hydrolysiert werden. Geringere Mengen als die angegebenen erfordern jedoch die Abtrennung von nicht umgesetztem Ausgangsprodukt, während größere Mengen als die angegebenen bei der Aufarbeitung die Salzfracht in unnötiger Weise erhöhen.

Das erfindungsgemäße Verfahren wird bei einer Temperatur von etwa 200 bis 350° C, bevorzugt 230 bis 350°C, besonders bevorzugt 255 bis 350°C und einem Druck von beispielsweise 10 bis 200 bar, abhängig von der verschieden hoch zu wählenden Reaktionstemperatur, durchgeführt. Der Druck, unter dem die Reaktion abläuft, ist im wesentlichen der Eigendruck der Komponente Wasser und t-Alkylamin im Reaktionsgemisch. Zusätzlich kann ein Fremddruck durch ein inertes Gas, wie $N_2$, $H_2$, $CH_4$ oder Edelgase, aufgedrückt werden. Dieser zusätzliche Druck ist jedoch nicht erfindungswesentlich. Bevorzugt wird unter dem Eigendruck des Reaktionssystems bei der eingestellten Reaktionstemperatur gearbeitet.

Zur Durchführung des erfindungsgemäßen Verfahrens kann beispielsweise das Ritter-Amid als

Feststoff oder als Schmelze mit der Alkalihydroxidlösung bei einer Temperatur zwischen Raumtemperatur und Reaktionstemperatur vermischt werden. Diese Vermischung kann diskontinuierlich oder kontinuierlich, bei Normaldruck oder bei erhöhtem Druck durchgeführt werden. Die Vermischung kann im Reaktionsdruckgefäß oder auch in einem vorgeschalteten Mischgefäß durchgeführt werden. Anschließend wird die Reaktionsmischung auf die angegebene Reaktionstemperatur gebracht und hierbei bis zur vollendeten Umsetzung belassen. Das Ende der Umsetzung ist beispielsweise daran kenntlich, daß der anfangs noch steigende Druck einen konstanten Wert erreicht. Während der Reaktion wird das Gemisch kräftig gerührt oder in einem Verweilzeitsystem in ständiger Bewegung gehalten, so daß eine ständige Durchmischung aller Reaktionspartner gewährleistet ist. Das erfindungsgemäße Verfahren kann grundsätzlich diskontinuierlich, beispielsweise in einem Autoklaven oder Druckkessel, oder auch kontinuierlich, beispielsweise in einem beheizbaren und druckfesten Strömungsrohr, durchgeführt werden.

Nach Beendigung der Reaktion wird das Reaktionsgemisch abgekühlt und entspannt. Danach wird das t-Alkylamin durch Destillation aus der Reaktionsmischung entfernt. Dieses Abdestillieren kann auch durch gezieltes Entspannen des noch heißen Reaktionsgemisches geschehen. Man erhält so gegebenenfalls nach einer weiteren Destillation zur Entfernung von Wasser ein t-Alkylamin mit einer Reinheit von größer als 97% in einer Ausbeute von mehr als 95% der theoretischen Ausbeute. Das erfindungsgemäß gewonnene t-Alkylamin ist frei von Ammoniak.

Höhersiedende t-Alkylamine, die nicht aus dem Reaktionsgemisch abdestilliert werden können, werden z. B. durch Extraktion mit einem geeigneten Extraktionsmittel aus dem Reaktionsgemisch isoliert und nach Entfernen des Extraktionsmittels z. B. durch Destillation gereinigt.

Als Destillationssumpf bzw. als extrahierte Wasserphase verbleibt eine wäßrige, alkalische Lösung des Natrium- oder Kalium-Salzes der Carbonsäure. Diese Lösung kann nach Neutralisation, beispielsweise durch Filtration oder Extraktion mit einem organischen Lösungsmittel von kleinen Resten nicht umgesetzten Ausgangsproduktes oder von Nebenprodukten gereinigt werden. In der Regel kann diese Lösung aber nach Einstellen des gewünschten Gehaltes an Carbonsäure-Salz durch Aufkonzentrieren oder Verdünnen direkt weiter verwendet werden.

Man kann auch aus den so erhaltenen Lösungen die freie Carbonsäure nach üblichen Methoden, beispielsweise durch Ansäuern der wäßrigen Phase Abtrennen der ausgefallenen Säure durch Filtration oder Extraktion isolieren. Dies sei am Beispiel des Phenylessigsäure-N-t-butylamids näher dargestellt:

Durch Umsetzung von Phenylessigsäure-N-t-butylamid z. B. mit wäßriger 15%iger Natronlauge oder 35%iger Kalilauge bei Temperaturen von z. B. 230 bis 280°C erhält man eine homogene Reaktionsmischung, aus der durch Abdestillieren t-Butylamin in hoher Reinheit und mehr als 95% Ausbeute isoliert werden kann.

Der verbleibende Destillationssumpf stellt nach einer entsprechenden Aufkonzentration durch Abdestillieren von Wasser eine gesättigte Lösung von Phenylessigsäure-Natrium-Salz oder Phenylessigsäure-Kalium-Salz dar.

Die im erfindungsgemäßen Verfahren anfallenden 10- bis 60%igen Lösungen der Alkali-, besonders der Kaliumsalze der Phenylessigsäure finden beispielsweise bei der Herstellung von Penicillin G durch Fermentation Verwendung (Ullmanns Enzyklopädie der technischen Chemie, 4. Auflage, 1976, Band 11, Seite 71 bis 72).

t-Butylamin kommt in vielen Bereichen zur Anwendung. So beschreibt FR 1 513 261 das t-Butylamin als wesentlichen Bestandteil fungizider Mischungen. Die DE-OS 2 006 830 beschreibt die Umsetzung von 1 Mol Dimethylamin und 1 Mol t-Butylamin in Gegenwart von wäßriger NaOH und Hexan bei 15 bis 30°C mit 2 Mol Schwefelmonochlorid zu einem Kautschuk-Vulkanisationsmittel. US 3 416 604 beschreibt die Verwendung von t-Butylamin als Vernetzerkomponente für Epoxidharze und FR 1 507 885 den Einsatz von t-Butylamin zur Herstellung von Celluloseacetat-Membranen.

Die im folgenden genannten Beispiele sollen das Verfahren näher beschreiben, jedoch nicht auf die genannten Beispiele einschränken.

## Beispiel 1

191,3 g (1 Mol) N-t-Butylphenylacetamid werden mit 300 g 15%iger NaOH (1,125 Mol) im 0,7-Ltr.-Nikkelautoklav unter kräftigem Rühren auf 260°C erhitzt. Nach 5 Stunden hat sich ein konstanter Druck von 53 bar eingestellt. Der Autoklav wird gekühlt und der Inhalt in eine Destillations-Apparatur gefüllt. Man destilliert über eine 30-cm-Kolonne bis zu einer Kopftemperatur von 100°C und erhält 73,4 g t-Butylamin mit einem Gehalt von 97,3%. Die Ausbeute beträgt 97,7% der theoretischen Ausbeute. Das t-Butylamin ist frei von Ammoniak.

Der Destillationssumpf (417 g) enthält 32,5 Gew.-% Phenylessigsäure (potentiometrische Titration nach Ionenaustausch) als Natriumsalz, entsprechend 99,5% der theoretischen Ausbeute.

Zur Isolierung der freien Phenylessigsäure wird der Destillationssumpf mit 300 ml 15%iger Salzsäure angesäuert, das ausfallende farblose, kristalline Produkt abgesaugt, mit 200 ml Wasser gewaschen und bei 50°C im Vakuum getrocknet.

Man erhält 131,7 g 97,5%ige Phenylessigsäure mit Fp. 76°C (Lit. Fp. 78°C) entsprechend einer Ausbeute von 94,3% der theoretischen Ausbeute.

## Beispiele 2—10

Analog Beispiel 1 wurden die Beispiele 2—10 ausgearbeitet, deren Reaktionsparameter und Ausbeuten in Tabelle 1 zusammengefaßt sind.

Tabelle 1

Alkalische Druckhydrolyse des N-t-Butylphenylacetamids (1 Mol)

| Bsp. Nr. | Mol Alkali | Konzentration des Alkali (%) | Reaktionstemperatur (°C) | Reaktionszeit (h) | Druck bar | Phenylessigsäure | | | t-Butylamin | | |
|---|---|---|---|---|---|---|---|---|---|---|---|
| | | | | | | Gewicht der Lösung (g) | Gehalt freie Säure (%) | % der theoretischen Ausbeute | Gewicht des Destillats | Gehalt (%) | % der theoretischen Ausbeute |
| 2 | 1,10 Mol NaOH | 14,5 | 260 | 3,0 | 51 | 418 | 32,2 | 99,0 | 75,0 | 95,6 | 98,0 |
| 3 | 1,00 Mol NaOH | 13,3 | 260 | 4,5 | 53 | 416 | 32,4 | 99,0 | 73,1 | 96,2 | 96,2 |
| 4 | 1,10 Mol KOH | 19,2 | 260 | 10 | 43 | 436 | 31,0 | 99,3[1]) | 73,7 | 96,4 | 97,1 |
| 5 | 1,00 Mol KOH | 17,75 | 260 | 13 | 79 | 432 | 31,2 | 98,9[1]) | 72,8 | 97,1 | 96,1 |
| 6 | 1,05 Mol NaOH | 30,0 | 250 | 5 | 49 | 446[2]) | 30,3 | 99,4 | 73,6 | 95,5 | 97,3 |
| 7 | 1,05 Mol NaOH | 48,0 | 250 | 5 | 47 | 405[2]) | 32,5 | 97,0 | 73,5 | 96,4 | 97,0 |
| 8 | 1,10 Mol NaOH | 15,0 | 230 | 3 | 43 | 437 | 30,7 | 98,7 | 74,3 | 96,7 | 98,4 |
| 9 | 1,10 Mol KOH | 35,0 | 230 | 7 | 43 | 298 | 45,2 | 99,1[1]) | 73,4 | 97,5 | 98,1 |
| 10 | 1,10 Mol KOH | 35,0 | 210 | 20 | 31 | 303 | 43,6 | 97,2[1]) | 73,2 | 97,1 | 97,3 |

[1]) Die K-Salzlösungen können ohne Zwischenisolierung für die Penicillinsynthese verwendet werden.
[2]) Nach Zugabe von 200 g Wasser.

Vergleichsbeispiel nach JP 50-95 210

145 g (0,5 Mol) N,N-Di-t-butyl-thiobispropionamid und 347 g 15%ige NaOH (1,3 Mol) werden 5 Stunden bei 180°C in einem 0,7 l Nickelautoklaven kräftig gerührt. Das erkaltete Reaktionsgemisch wird in eine Destillationsapparatur gefüllt. Bis zu einer Kopftemperatur von 100°C wird das Amin abdestilliert. Man erhält 55,5 g t-Butylamin mit einem Gehalt von 97,3%. Ausbeute: 74% der theoretischen Ausbeute.

Der Destillationssumpf wird mit 30%iger Salzsäure auf pH = 1 gestellt. Dabei treten große Mengen Schwefelwasserstoff aus dem Reaktionsgemisch aus. Die ausgefallene Carbonsäure wird abgesaugt, mit 100 ml Wasser gewaschen und getrocknet. Man erhält 53,3 g Thiobispropionsäure mit einem Gehalt von 83%. Ausbeute: 49,7% der theoretischen Ausbeute.

Beispiel 11

225,5 g (1 Mol) N-t-Butyl-(2-chlorphenyl)-acetamid (Fp: 128—130°C) werden mit 281 g 15%iger NaOH (1,05 Mol) im 0,7-l-Nickelautoklav unter Rühren 3 h auf 260°C erhitzt. Der Autoklav wird gekühlt und die Reaktionsmischung in eine Destillationsapparatur gefüllt. Man destilliert über eine 30-cm-Kolonne bis zu einer Kopftemperatur von 100°C und erhält 71,5 g t-Butylamin mit einem Gehalt von 97%, entsprechend einer Ausbeute von 95% der theoretischen Ausbeute. Der Destillationssumpf wird mit 200 ml Wasser und 100 ml 30%iger Salzsäure versetzt. Die ausgefallene Säure wird abgesaugt und im Vakuum getrocknet. Man erhält 157,8 g o-Chlorphenylessigsäure mit Fp: 95°C. Ausbeute: 92% der theoretischen Ausbeute.

Beispiel 12

Analog Beispiel 11 werden 260 g (1 Mol) N-t-Butyl-(2,4-dichlorphenyl)-acetamid (Fp: 111—113°C) mit 281 g 15%iger NaOH (1,05 Mol) bei 250° umgesetzt. Man erhält nach Destillation 72,8 g t-Butylamin mit einem Gehalt von 97,8%, entsprechend 97,3% Ausbeute.

Durch Ansäuern des Destillationssumpfes erhält man 182,2 g 2,4-Dichlorphenylessigsäure mit Fp: 116—118°C. Ausbeute: 89% der theoretischen Ausbeute.

Beispiel 13

Analog Beispiel 11 werden 205 g (1 Mol) N-t-Pentylphenylacetamid (Fp: 96°C) mit 293 g 15%iger NaOH (1,1 Mol) fünf Stunden bei 255°C gerührt. Man erhält nach Destillation 85,2 g t-Pentylamin mit einem Gehalt von 97%, entsprechend einer Ausbeute von 95%.

Durch Ansäuern des Destillationssumpfes erhält man 123 g Phenylessigsäure. Ausbeute 90,4%.

Beispiel 14

191,2 g (1 Mol) N-t-Butyl-p-methylbenzamid (Fp: 113—115°C) werden mit 281 g 15%iger NaOH (1,05 Mol) fünf Stunden bei 250°C gerührt. Die Reaktionsmischung wird in eine Destillationsapparatur gefüllt und das Amin über eine Kolonne abdestilliert: Man erhält 72 g t-Butylamin mit einem Gehalt von 97,4%, entsprechend einer Ausbeute von 96%.

Der Destillationssumpf wird mit 130 ml 30%iger Salzsäure angesäuert und das ausgefallene Produkt abgesaugt und getrocknet. Man erhält 124,2 g p-Toluyl-Säure vom Fp 178—179°C. Ausbeute: 91,3%.

Beispiel 15

158,6 g (0,75 Mol) o-Chlorbenzoesäure-N-t-butylamid (Fp: 104—105°C) werden mit 220 g 15%iger NaOH (0,825 Mol) für zehn Stunden unter kräftigem Rühren auf 280°C erhitzt. Die Reaktionsmischung wird in eine Destillationsapparatur gefüllt und das Amin abdestilliert. Man erhält 50,5 g t-Butylamin mit einem Gehalt von 97,1%. Der Destillationssumpf wird mit 10%iger Salzsäure auf pH = 7 gestellt und der Niederschlag abgesaugt und mit 100 ml Wasser gewaschen. Nach Trocknung erhält man 10,6 g restliches o-Chlorbenzoesäure-N-t-butylamid. Daraus ergibt sich ein Umsatz von 93,3%.

Die neutrale Mutterlauge und das Waschwasser werden vereinigt und mit 100 ml 30%iger Salzsäure auf pH = 1 gestellt. Die ausgefallene Säure wird abgesaugt und getrocknet. Man erhält 95,4 g o-Chlorbenzoesäure vom Fp 136—138°C. Ausbeute: 86%.

Beispiel 16

191,0 g (1 Mol) Benzoesäure-N-t-pentylamid werden mit 281 g 15%iger NaOH (1,05 Mol) unter Rühren 4 h auf 250° C erhitzt. Aufarbeitung wie in Beispiel 15 liefert 86,6 g t-Pentylamin mit einem Gehalt von 97,2%, entsprechend 96,7% Ausbeute und 116 g Benzoesäure, entsprechend 95% Ausbeute.

Beispiel 17

227 g (1 Mol) $\alpha$-Naphthoesäure-N-t-butylamid (Fp: 144—146° C) werden mit 293 g 15%iger NaOH (1,10 Mol) 10 Stunden bei 280° C kräftig gerührt. Die erkaltete Reaktionsmischung wird in eine Destillationsapparatur übergeführt und bis zu einer Sumpftemperatur von 105° C das Amin abdestilliert. Man erhält 71,6 g t-Butylamin mit einem Gehalt von 98,2%. Ausbeute: 96,3%.

Der Destillationssumpf wird auf pH = 1 gestellt und die ausgefallene Säure isoliert und getrocknet. Man erhält 160 g $\alpha$-Naphthoesäure mit Fp: 162—163°. Ausbeute: 92,9%.

Beispiel 18

178,1 g (1 Mol) Isonicotinsäure-N-t-butylamid (Fp: 119—123°) werden mit 266 g 15%iger Natronlauge (1 Mol) 4 Stunden bei 260° C gerührt. Es stellt sich ein Druck von 45 bar ein. Aus dem Reaktionsgemisch wird bis zu einer Kopftemperatur von 100° C das Amin abdestilliert. Man erhält 71,5 g t-Butylamin mit einem Gehalt von 97%. Ausbeute 95%.

Der Destillationssumpf wird auf pH = 3 eingestellt und die ausgefallene Säure abgesaugt, mit 100 ml Wasser gewaschen und getrocknet. Man erhält 112 g Isonicotinsäure mit Fp > 300° C. Ausbeute: 91%.

Beispiel 19

178,1 g (1 Mol) N-t-Butyl-picolinsäureamid ($Kp_{10}$: 150° C) werden mit 293 g 15%iger NaOH (1,1 Mol) zwei Stunden bei 250° C gerührt und wie in Beispiel 19 aufgearbeitet. Man erhält 71,7 g t-Butylamin mit einem Gehalt von 97,4%. Ausbeute: 95,6%.

Der Destillationssumpf wird mit 30%iger Salzsäure auf pH = 3 gestellt, die ausgefallene Säure wird abgesaugt und getrocknet. Man erhält 108 g Picolinsäure mit Fp. 134—136° C. Ausbeute: 88%.

**Patentansprüche**

1. Verfahren zur gleichzeitigen Herstellung von Carbonsäuren und N-tert.-Alkylaminen, dadurch gekennzeichnet, daß man ein Carbonsäure-N-t-alkylamid der Formel

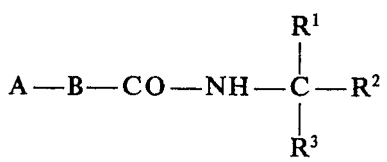

in der

A für einen gegebenenfalls substituierten Phenyl-, Naphthyl- oder Pyridylrest oder den aliphatischen Rest

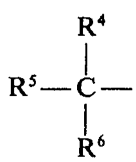

steht, wobei $R^4$, $R^5$ und $R^6$ gleich oder verschieden sind und unabhängig voneinander Wasserstoff oder geradkettige oder verzweigte Alkylgruppen mit 1 bis 8 Kohlenstoffatomen bedeuten, wobei weiterhin $R^4$ und $R^5$ gemeinsam einen aliphatischen Ring von 4 bis 8 Ringgliedern bilden können,

B für eine Kohlenstoff-Einfachbindung, für einen aliphatischen Rest mit 1 bis 6 Kohlenstoffatomen oder einen cycloaliphatischen Rest mit 4 bis 8 Kohlenstoffatomen steht, wobei jedes Kohlenstoffatom unabhängig von den anderen mit geradkettigen oder verzweigten $C_1—C_4$-Alkylgruppen substituiert sein kann, und

$R^1$, $R^2$, $R^3$ gleich oder verschieden sind und unabhängig voneinander geradkettige oder verzweigte Alkylgruppen mit 1 bis 8 Kohlenstoffatomen bedeuten, wobei weiterhin $R^1$ und $R^2$ gemeinsam einen aliphatischen Ring von 4 bis 8 Ringgliedern bilden können oder $R^1$ ein gegebenenfalls substituierter Phenylrest sein kann,

mit 5- bis 50gew.-%igem wäßrigem Alkalihydroxid in einer Menge von 1,0 bis 1,3 Mol pro Mol des Amids bei 200 bis 350° C unter erhöhtem Druck umsetzt.

2. Verfahren nach Anspruch 1, dadurch gekennzeichnet, daß man ein Carbonsäure-N-t-alkylamid der Formel

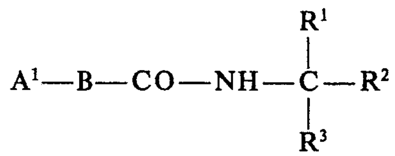

umsetzt, wobei

$A^1$ den gegebenenfalls substituierten Phenyl-, Naphthyl- oder Pyridylrest bedeutet und
B, $R^1$, $R^2$ und $R^3$ die in Anspruch 1 angegebene Bedeutung haben.

3. Verfahren nach Anspruch 1, dadurch gekennzeichnet, daß man ein Carbonsäure-N-t-alkylamid der Formel

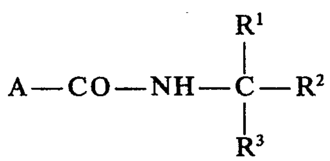

umsetzt, wobei

A, $R^1$, $R^2$ und $R^3$ die in Anspruch 1 angegebene Bedeutung haben.

4. Verfahren nach Anspruch 1, dadurch gekennzeichnet, daß man ein Carbonsäure-N-t-alkylamid der Formel

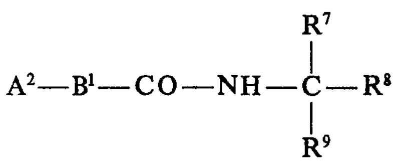

umsetzt, wobei

$A^2$ den gegebenenfalls substituierten Phenylrest,
$B^1$ eine Kohlenstoff-Einfachbindung oder eine $CH_2$-Gruppe, die mit geradkettigen und/oder verzweigten $C_1-C_4$-Alkylgruppen substituiert sein kann, und
$R^7$, $R^8$ und $R^9$ unabhängig voneinander Methyl oder Ethyl bedeuten.

5. Verfahren nach Anspruch 1 bis 4, dadurch gekennzeichnet, daß 10- bis 35gew.-%iges Alkalihydroxid eingesetzt wird.

6. Verfahren nach Anspruch 1 bis 5, dadurch gekennzeichnet, daß 1,0 bis 1,15 Mol Alkalihydroxid pro Mol Amid eingesetzt wird.

7. Verfahren nach Anspruch 1 bis 5, dadurch gekennzeichnet, daß 1,0 bis 1,05 Mol Alkalihydroxid pro Mol Amid eingesetzt wird.

8. Verfahren nach Anspruch 1 bis 7, dadurch gekennzeichnet, daß bei 230 bis 350° C gearbeitet wird.

9. Verfahren nach Anspruch 1 bis 7, dadurch gekennzeichnet, daß bei 255 bis 350° C gearbeitet wird.

10. Verfahren nach Anspruch 1 bis 9, dadurch gekennzeichnet, daß bei dem Eigendruck des Reaktionssystems gearbeitet wird.

**Claims**

1. Process for the simultaneous preparation of carboxylic acids and N-tert.-alkylamines, characterised in that a carboxylic acid N-t.-alkylamide of the formula

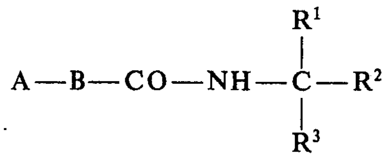

in which

A represents an optionally substituted phenyl, naphthyl or pyridyl radical or the aliphatic radical

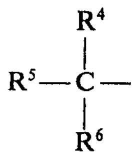

wherein $R^4$, $R^5$ and $R^6$ are identical or different and independently of one another denote hydrogen or straight-chain or branched alkyl groups having 1 to 8 carbon atoms, it being also possible for $R^4$ and $R^5$ together to form an aliphatic ring having 4 to 8 ring members,

B represents a single carbon bond, an aliphatic radical having 1 to 6 carbon atoms or a cycloaliphatic radical having 4 to 8 carbon atoms, it being possible for each carbon atom, independently of the others, to be substituted by straight-chain or branched $C_1-C_4$-alkyl groups, and

$R^1$, $R^2$ and $R^3$ are identical or different and independently of one another denote straight-chain or branched alkyl groups having 1 to 8 carbon atoms, it being also possible for $R^1$ and $R^2$ together to form an aliphatic ring having 4 to 8 ring members or for $R^1$ to be an optionally substituted phenyl radical,

is reacted at 200 to 350°C under elevated pressure with 5 to 50% strength by weight aqueous alkali metal hydroxide in an amount of 1.0 to 1.3 mols per mol of the amide.

2. Process according to Claim 1, characterised in that a carboxylic acid N-t.-alkylamide of the formula

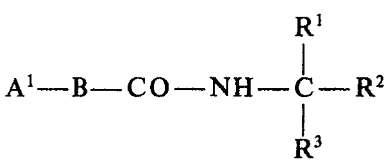

wherein

$A^1$ denotes the optionally substituted phenyl, naphthyl or pyridyl radical and
B, $R^1$, $R^2$ and $R^3$ have the meaning indicated in Claim 1,

is reacted.

3. Process according to Claim 1, characterised in that a carboxylic acid N-t.-alkylamide of the formula

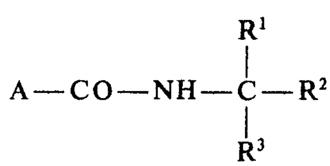

wherein

A, $R^1$, $R^2$ and $R^3$ have the meaning indicated in Claim 1,

is reacted.

4. Process according to Claim 1, characterised in that a carboxylic acid N-t.-alkylamide of the formula

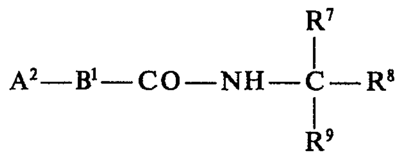

wherein

$A^2$ denotes the optionally substituted phenyl radical,
$B^1$ denotes a single carbon bond or a $CH_2$ group which can be substituted by straight-chain and/or branched $C_1-C_4$-alkyl groups, and
$R^7$, $R^8$ and $R^9$ independently of one another denote methyl or ethyl,

is reacted.

5. Process according to Claim 1 to 4, characterised in that 10 to 35% strength by weight alkali metal hydroxide is employed.

6. Process according to Claim 1 to 5, characterised in that 1.0 to 1.15 mols of alkali metal hydroxide are employed per mol of amide.

7. Process according to Claim 1 to 5, characterised in that 1.0 to 1.05 mols of alkali metal hydroxide are employed per mol of amide.

8. Process according to Claim 1 to 7, characterised in that the reaction is carried out at 230 to 350° C.

9. Process according to Claim 1 to 7, characterised in that the reaction is carried out at 255 to 350° C.

10. Process according to Claim 1 to 9, characterised in that the reaction is carried out under the autogenous pressure of the reaction system.

**Revendications**

1. Procédé pour la fabrication simultanée d'acides carboxyliques et de N-tert.-alkylamines, caractérisé en ce que l'on fait réagir un N-t-alkylamide d'acide carboxylique de formule

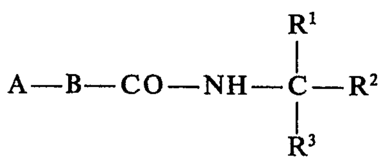

dans laquelle

A représente un reste phényle, naphtyle ou pyridyle éventuellement substitué ou le reste aliphatique

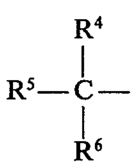

dans lequel $R^4$, $R^5$ et $R^6$ sont identiques ou différents et représentent indépendamment l'hydrogène ou des groupes alkyles à chaîne droite ou ramifiée en $C_1-C_8$, $R^4$ et $R^5$ pouvant en outre former ensemble un noyau aliphatique ayant 4 à 8 chaînons,

B représente une liaison carbonée simple, un reste aliphatique en $C_1-C_6$ ou un reste cycloaliphatique en $C_4-C_8$, chaque atome de carbone pouvant être substitué indépendamment des autres par des groupes alkyles à chaîne droite ou ramifiée en $C_1-C_4$, et

$R^1$, $R^2$ et $R^3$ sont identiques ou différents et représentent indépendamment l'un de l'autre des groupes alkyles à chaîne droite ou ramifiée en $C_1-C_8$, $R^1$ et $R^2$ pouvant en outre former ensemble un cycle aliphatique ayant de 4 à 8 chaînons, ou bien $R^1$ pouvant être un reste phényle éventuellement substitué,

avec un hydroxyde alcalin aqueux à 5—50% en poids en quantité de 1,0 à 1,3 mole par mole de l'amide, à 200—350° C sous pression élevée.

2. Procédé selon la revendication 1, caractérisé en ce que l'on fait réagir un N-t-alkylamide d'acide

carboxylique de formule

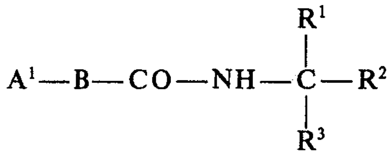

dans laquelle

$A^1$ représente le reste phényle, naphtyle ou pyridyle éventuellement substitué et
B, $R^1$, $R^2$ et $R^3$ ont la signification indiquée à la revendication 1.

3. Procédé selon la revendication 1, caractérisé en ce que l'on fait réagir un N-t-alkylamide d'acide carboxylique de formule

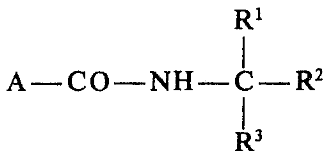

dans laquelle

A, $R^1$, $R^2$ et $R^3$ ont la signification indiquée dans la revendication 1.

4. Procédé selon la revendication 1, caractérisé en ce que l'on fait réagir un N-t-alkylamide d'acide carboxylique de formule

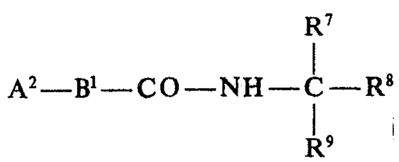

dans laquelle

$A^2$ représente le reste phényle éventuellement substitué
$B^1$ représente une liaison simple carbonée ou un groupe $-CH_2$ qui peut être substitué par des groupes alkyles à chaîne droite et/ou ramifiés en $C_1-C_4$, et
$R^7$, $R^8$ et $R^9$ représentent indépendamment l'un de l'autre méthyle ou éthyle.

5. Procédé selon les revendications 1 à 4, caractérisé en ce que l'on utilise un hydroxyde alcalin à 10—35% en poids.

6. Procédé selon les revendications 1 à 5, caractérisé en ce que l'on utilise 1,0 à 1,15 mole d'hydroxyde alcalin par mole d'amide.

7. Procédé selon les revendications 1 à 5, caractérisé en ce que l'on utilise 1,0 à 1,05 mole d'hydroxyde alcalin par mole d'amide.

8. Procédé selon les revendications 1 à 7, caractérisé en ce que l'on opère à 230—350° C.

9. Procédé selon les revendications 1 à 7, caractérisé en ce que l'on opère à 255—350° C.

10. Procédé selon les revendications 1 à 9, caractérisé en ce que l'on opère sous la pression propre du système de réaction.